# EUROPÄISCHE PATENTSCHRIFT

(11) **EP 0 656 424 B1**
(45) Veröffentlichungstag und Bekanntmachung des Hinweises auf die Patenterteilung: **06.02.2002**
(21) Anmeldenummer: 94890199.6
(22) Anmeldetag: 30.11.1994
(51) Int. Cl.: C12Q 1/56, G01N 33/86

(54) **Test zur Bestimmung der Empfindlichkeit gegenüber aktiviertem Protein C**
Assay to determine the sensitivity to activated protein C
Essai pour déterminer la sensibilité à la protéine C activée

(30) Priorität: 03.12.1993 US 160877
(43) Veröffentlichungstag der Anmeldung: 07.06.1995
(73) Patentinhaber: Baxter Aktiengesellschaft, 1221 Wien (AT)
(72) Erfinder: Varadi, Katalin, Dr., A-1230 Wien (AT); Schwarz, Hans Peter, Doz., A-1180 Wien (AT); Lang, Hartmut, Dr., A-2560 Grillenberg (AT); Moritz, Berta, Dr., A-1030 Wien (AT)
(74) Vertreter: Weinzinger, Arnulf, Dipl.-Ing.

(56) Entgegenhaltungen:
- EP-A- 0 434 377
- EP-A- 0 496 723
- WO-A-91/01382
- WO-A-93/10261
- WO-A-93/10262
- THROMBOSIS RESEARCH, Bd. 75, Nr. 4, 15.August 1994 NEW YORK US, Seiten 395-400, M. BOKAREWA ET AL. 'Heterogeneity of the APC-resistance phenomenon'
- THE JOURNAL OF BIOLOGICAL CHEMISTRY, Bd. 269, Nr. 29, 22.Juli 1994 BALTIMORE US, Seiten 18735-18738, L. SHEN ET AL. 'Factor V and protein S as synergistic cofactors to activated protein C in degradation of Factor VIIIa'
- BRITISH JOURNAL OF HAEMATOLOGY, Bd. 90, Nr. 4, August 1995 OXFORD GB, Seiten 884-891, K. VARADI ET AL. 'A chromogenic assay for activated protein C resistance'

## Beschreibung

Die vorliegende Erfindung betrifft einen verbesserten Test zur Bestimmung der Auswirkungen von aktiviertem Protein C ("APC") in Testproben zur Gewährleistung rascher und genauer Auswertungen. Eine Testprobe inkludiert jegliches Material, von dem erwartet wird, daß es eine Wechselwirkung mit aktiviertem Protein C eingehen kann. Diese Materialien sind typischerweise Plasma oder Plasmafraktionen.

Der vorliegende Test beruht auf der Messung der Umwandlung von Faktor X in aktivierten Faktor X ("Faktor Xa") durch aktivierten Faktor VIII ("Faktor VIIIa"), der in der Testlösung vorhanden ist. APC inhibiert diese Umwandlung.

Protein C ist ein Vitamin K-abhängiges Glycoprotein, das in der Leber synthetisiert wird und im Plasma als inaktives Zymogen in einer Konzentration von etwa 4 µg/ml zirkuliert. Dieses Protein wird durch den Thrombin-Thrombomodulin-Komplex an der Oberfläche der Gefäßwand (Endothelium) in eine aktive Serin-Protease (aktiviertes Protein C, APC) umgewandelt. Protein C kann auch durch nicht-physiologische Enzyme, wie den Schlangengift-Faktor (Protac C®, Pentapharm, Schweiz), aktiviert werden.

APC hat aufgrund seiner Fähigkeit, den Plasminogen-Aktivator-Inhibitor zu inaktivieren, fibrinolytische Eigenschaften. APC hat auch eine Antikoagulationswirkung, da es Faktor Va und Faktor VIIIa proteolytisch abbauen kann. Faktor Va ist ein Cofaktor für die Faktor Xa-induzierte Aktivierung von Prothrombin zu Thrombin. Faktor VIIIa ist der Cofaktor der Umwandlung von Faktor X zu Faktor Xa. Wie oben erwähnt, ist Faktor Xa zusammen mit Faktor Va an der Umwandlung von Prothrombin zu Thrombin beteiligt. Demgemäß stellt die Aktivierung von Protein C *in vivo* zur Bildung von APC eine negative Feedback-Reaktion bei der Erzeugung von Thrombin auf dem Weg über den Abbau der Faktoren Va und VIIIa dar.

Die Empfindlichkeit eines Individuums gegenüber der normalen Aktivität von aktiviertem Protein C auf die Inaktivierung von Faktor VIIIa ist als "APC-Empfindlichkeit" oder "APC-response" definiert. Die APC-Empfindlichkeit kann mittels Testproben, wie Plasmaproben oder Fraktionen, die von einem bestimmten Individuum stammen, bestimmt werden. Plasmaproben oder -fraktionen von Individuen, die gegenüber APC sehr empfindlich sind, weisen bei Zugabe von APC eine starke Antikoagulationsaktivität auf. Plasmaproben von Individuen mit verminderter APC-Empfindlichkeit weisen eine minimale Antikoagulationsaktivität auf.

Zur Entwicklung der optimalen APC-Aktivität ist ein Cofaktor, Protein S, notwendig. Protein S ist ein nicht-enzymatischer Cofaktor für die Antikoagulations- und Pro-Fibrinolyse-Eigenschaften von APC. Im Plasma ist Protein S in verschiedenen Formen vorhanden. Zu diesen Formen gehört eine freie, aktive Form und eine inaktive Form, die mit dem C4b-binding-Protein nicht-covalent komplexiert ist. In einem Protein-S-verarmten Plasma kann APC seine normale Aktivität nicht ausüben. Nach Zugabe von Protein S erlangt das APC seine normale Aktivität wieder.

APC erhöht die Gerinnungszeit des Plasmas in Abhängigkeit von der Dosis. Die pathophysiologische Rolle von APC wird an Individuen demonstriert, die an durch einen angeborenen Protein S- oder Protein C-Mangel verursachter Thrombophilie leiden. Der angeborene Protein S-Mangel wird autosomal-dominant vererbt und ist durch das Auftreten venöser und arterieller Thromboembolien in früher Jugend gekennzeichnet. Andere durch APC-Abnormitäten verursachte negative Wirkungen umfassen mikrovaskuläre Koagulation, Reperfusionsverletzungen und septischen Schock. Vgl. Esmon, *TCM* 2: 214-19 (1992).

Für die verringerte APC-Empfindlichkeit kann eine Vielfalt von Ursachen verantwortlich sein. Beispielsweise entdeckten Dahlbäck *et al.* ein Syndrom, das durch eine schwache Antikoagulationsreaktion auf APC gekennzeichnet ist. Vgl. *Proc. Nat'l Acad. Sci. USA* 90: 1004-08 (1993). Es zeigte sich, daß das Blut von Patienten mit diesem Syndrom normale Mengen an Antithrombin III, Protein C, Protein S, Plasminogen, Fibrinogen und anderen Koagulationsfaktoren aufwies. Dahlbäck *et al*. postulierten, daß ein zuvor unerkannter Cofaktor von APC im Plasma von Patienten mit diesem Syndrom fehlte. Man glaubt, daß dieses Syndrom ererbt wird. Verläßliche Tests zur Messung des APC-Cofaktors sind aufgrund des Vorhandenseins solcher Syndrome notwendig.

Zur Beurteilung der Blutkoagulationsstörungen eines Individuums wurden bereits früher Tests entwickelt. Einer dieser Tests ist in der WO 93/10261 beschrieben und beruht auf der Kombination eines Aktivators des Blutgerinnungssystems mit APC und Hilfsmitteln, wie Phospholipiden und Calciumionen. Bei diesem Verfahren wird die Antikoagulationsreaktion auf menschliches APC mit Koagulationstests auf Basis von Faktor IXa und Faktor Xa ausgewertet. Es heißt, daß dieser Test das Auffinden von Krankheiten, die nicht durch Protein S-Mängel oder von APC-resistentem Faktor Va und VIIIa verursacht sind, ermöglicht.

Der Test aus der WO 93/10261 betrifft die gesamte Koagulationskaskade und benützt mehr als einen Inkubationsschritt. Dieser Test erfordert hohe Plasmakonzentrationen, da er den Beginn der Koagulationskaskade (Kallikrein) involviert. Diese Konzentrationen sind mehr als 10% V/V Plasmaprobe, üblicherweise sogar 20-35% V/V. Die hohen Plasmakonzentrationen in der Reaktionsmischung machen es möglich, daß die Faktoren innerhalb der Probe den Test stören und dadurch seine Empfindlichkeit und Spezifität verringern.

Faktor VIII-Aktivitätstests sind bei der Auswertung der APC-Empfindlichkeit bis zu einem gewissen Grad anwendbar. Ein solcher Test ist in der EP-A-0 496 723 beschrieben. Das bei diesem Test verwendete Reagens enthält Faktor IXaβ, Faktor X, Calciumionen, Thrombin, Phospholipide und gewünschtenfalls Faktor XIa und Faktor XIIa. Dieser Test beruht auf der Aktivierung von Faktor X infolge von in der Probe enthaltenem Faktor VIII. Faktor Xa wird dann quantitativ bestimmt.

Wenn dieser Test zur Auswertung von Proteinen, die mit Faktor VIII reagieren, wie APC und sein Cofaktor Protein S, verwendet wird, wird eine Überschußmenge an Faktor VIII zur Probe zugegeben und umgesetzt. Die Reaktion verringert die Faktor VIII-Aktivität, und die verbleibende Menge an Faktor VIII wird dann unter Verwendung eines chromogenen Reagens für Faktor VIII bestimmt. Es ist jedoch im Zusammenhang mit der Bestimmung der APC-Empfindlichkeit erwünscht, das Verhältnis der diese Aktivität beeinflussenden Substanzen, wie Faktor VIII, ähnlich den im Plasma gefundenen Mengen aufrechtzuerhalten. Außerdem erfordert dieses Verfahren im Zusammenhang mit der APC-Empfindlichkeit mehr als einen Inkubationsschritt.

### Zusammenfassung der Erfindung

Ein Ziel der vorliegenen Erfindung ist die Schaffung eines verbesserten Tests zur Bestimmung der APC-Empfindlichkeit eines Individuums.

Auch ein Ziel der vorliegenden Erfindung ist es, einen verbesserten Test zur Bestimmung der APC-Empfindlichkeit zur Verfügung zu stellen, der keine Zugabe von Faktor VIII zu einer Testprobe erfordert.

Noch ein Ziel der vorliegenden Erfindung ist die Schaffung eines verbesserten Tests zur Bestimmung der APC-Empfindlichkeit, der nur einen Inkubationsschritt erfordert.

Ein weiteres Ziel der vorliegenden Erfindung ist die Schaffung eines verbesserten Tests zur Bestimmung der APC-Empfindlichkeit, der nicht die gesamte Koagulationskaskade involviert.

Noch ein weiteres Ziel der vorliegenden Erfindung ist es, einen verbesserten Test zur Bestimmung der APC-Empfindlichkeit zur Verfügung zu stellen, der keine Bestimmung aller in einer Testprobe vorhandenen Blutgerinnungsfaktoren erfordert.

Noch ein weiteres Ziel der vorliegenden Erfindung ist die Schaffung eines verbesserten Tests zur Bestimmung der APC-Empfindlichkeit, der minimale Mengen an Testprobe zur Durchführung eines Tests erfordert.

Zur Erreichung dieser Ziele ist gemäß einem Aspekt der vorliegenden Erfindung ein Test zur Bestimmung der Empfindlichkeit einer Testprobe, wie einer Plasmaprobe oder -fraktion, gegenüber aktiviertem Protein C vorgesehen. Gemäß einem Aspekt der vorliegenden Erfindung werden für den Test aktivierter Faktor IX ("Faktor IXa"), Faktor X, Thrombin, APC, Calciumionen und Phospholipide verwendet. Vorzugsweise liegt der Faktor IXa in der reifen Form, als Faktor IXaß bekannt, vor. Die Menge an gemäß dem vorliegenden Test verwendetem Plasma braucht nicht mehr als 10% V/V zu sein. Bei einer bevorzugten Ausführungsform beträgt die im Test verwendete Plasmamenge weniger als 2%, und bei einer bevorzugteren Ausführungsform beträgt die Plasmamenge 0,8-1,6%.

Gemäß einem anderen Aspekt der vorliegenden Erfindung ist ein Verfahren zur Bestimmung der Empfindlichkeit gegenüber aktiviertem Protein C in einer Testprobe vorgesehen, welches die Schritte (a) der Umsetzung von Faktor X, aktiviertem Faktor IX, aktiviertem Protein C und Thrombin mit der Testprobe in einer Calciumionen und Phospholipide enthaltenden Testlösung, wobei die Testprobe höchstens 10% V/V der Testlösung ausmacht, (b) der Bestimmung der Menge an in der Reaktion von Schritt (a) gebildetem aktivierten Faktor X, und die Berechnung der Empfindlichkeit der Testprobe gegenüber aktiviertem Protein C auf Basis der Bestimmung von Schritt (b) im Vergleich zu einer Kontrolle umfaßt. Es ist bevorzugt, daß bei der Kontrolle die Testprobe mit aktiviertem Faktor IX, Faktor X, Thrombin, Calciumionen und Phospholipiden ohne Zugabe (d.h. in Abwesenheit) von aktiviertem Protein C umgesetzt wird. Die Kontrolle kann auch auf bekannten Informationen, wie einer historischen Kontrolle aufgrund von er-stellten Bereichen von Blutkomponenten und Aktivitäten, basieren.

Zur Durchführung der vorliegenden Erfindung kann ein Testset zur Bestimmung der Empfindlichkeit einer Testprobe gegenüber aktiviertem Protein C vorgesehen werden, welches eine erste und eine zweite Reagenskomponente umfaßt, wobei die erste Reagenskomponente Faktor IXa und Faktor X umfaßt und die zweite Reagenskomponente aktiviertes Protein C umfaßt. Thrombin, Calciumionen und Phospholipide sind entweder in der ersten Reagenskomponente oder in der zweiten Reagenskomponente vorhanden.

Gemäß einer bevorzugten Ausführungsform umfaßt die erste Reagenskomponente Calciumionen und die zweite Reagenskomponente Phospholipide, wobei wahlweise die erste oder die zweite Reagenskomponente Thrombin umfaßt.

Zur Durchführung der vorliegenden Erfindung kann ein Testset zur Bestimmung der Empfindlichkeit einer Testprobe gegenüber aktiviertem Protein C vorgesehen werden, welches eine erste, eine zweite und eine dritte Reagenskomponente umfaßt, wobei die erste Reagenskomponente Faktor IXa, Faktor X und Calciumionen umfaßt; die zweite Reagenskomponente aktiviertes Protein C umfaßt; und die dritte Reagenskomponente Phospholipide umfaßt. Thrombin ist ebenfalls in der ersten oder in der dritten Reagenskomponente vorhanden.

Zur erfindungemäßen Bestimmung der Empfindlichkeit einer Testprobe gegenüber aktiviertem Protein C kann ein Testreagens vorgesehen werden, welches Faktor IXa, Faktor X, Thrombin, aktiviertes Protein C, Phospholipide und Calciumionen umfaßt. Vorzugsweise enthält das Testreagens keinen Faktor VIII.

### Detaillierte Beschreibung bevorzugter Ausführungsformen

Seit langem ist der Bedarf an einem verläßlichen und genauen Test zur Bestimmung der APC-Empfindlichkeit eines Individuums erkannt. Individuen mit abnormaler APC-Empfindlichkeit sind für Erkrankungen, wie Thrombosen und thromboembolische Erkrankungen anfällig. Eine abnormale APC-Empfindlichkeit kann durch einen Mangel an Protein S, einen Mangel oder einen Überschuß an APC-Inhibitoren oder Cofaktoren, und den APC-resistenten Faktor Va oder Faktor VIIIa verursacht sein. Da eine abnormale APC-Empfindlichkeit durch eine Vielfalt von Defekten in der Blutkoagulationskaskade verursacht sein kann, sollte der verbesserte Test die APC-Empfindlichkeit unabhängig von der Ursache feststellen können.

Der vorliegende Test für APC-Empfindlichkeit beruht auf der Aktivierung von Faktor X zu Faktor Xa. In Anwesenheit von Faktor IXa und Faktor VIIIa wird Faktor X zu Faktor Xa aktiviert. Faktor Xa kann durch Verwendung eines chromogenen Substrats für Faktor Xa nachgewiesen werden. Die Anwesenheit von Faktor Xa kann auch durch die Umwandlung von Prothrombin zu Thrombin oder durch Messung der Koagulationszeit bestimmt werden.

Beim vorliegenden Test wird eine Testprobe, wie eine Plasmaprobe oder -fraktion, zu einer gereinigten Faktor IXa, Faktor X, Thrombin, APC, Calciumionen und Phospholipide enthaltenden Lösung zugegeben. Vorzugsweise wird in diesem Test die reife Form von Faktor IXa, als Faktor IXaβ bekannt, verwendet. Faktor IXa, Faktor X, Thrombin, APC, Calciumionen und Phospholipide können zur Bildung eines "Testreagens" vereinigt werden, zu welchem die Testprobe zur Bildung einer "Testlösung" zugegeben wird. Faktor VIII wird der Testlösung nicht zugesetzt; statt dessen stützt man sich auf den bereits in der Testprobe vorhandenen Faktor VIII. Typischerweise können diese Materialien des Testreagens als zwei oder mehrere "Reagenskomponenten" vorverpackt sein und dann später direkt vor der Zugabe der Testprobe vereinigt werden. Nach der Schaffung des Testreagens kann die Bestimmung einer Testprobe nach einem einzigen Inkubationsschritt durchgeführt werden.

Ein im erfindungsgemäßen Verfahren verwendbares Reagens enthält vorzugsweise Faktor IXaβ in einem Bereich von 0,05 und 5 E/ml, Faktor X in einem Bereich von 0,01 und 10 E/ml, Thrombin in einer Konzentration im Bereich zwischen 0,01 und 2,0 E/ml, Phospholipide in einem Bereich von 0,01 bis 100 nmol/ml, Calciumionen in einem Bereich von 1 bis 50 µmol/ml, APC in einem Bereich von 0,05-5 E/ml und gegebenenfalls Protein S in einer Konzentration, die dem Verhältnis von 1 bis 10 Einheiten, vorzugsweise 3 bis 5 Einheiten, pro ml unverdünnter Testprobe entspricht.

Bevorzugt werden menschliche Blutgerinnungsfaktoren, Thrombin und aktiviertes Protein C verwendet. Es ist weiters bevorzugt, daß diese Komponenten Inaktivierungsverfahren gegen infektiöse Agentien unterzogen worden sind.

Zur Durchführung des erfindungsgemäßen Testverfahrens reichen bereits geringe, aber noch meßbare Spuren an Thrombin in der Testlösung bzw. im Testreagens aus. Einige kommerziell erhältliche Reagenzien zur Bestimmung der Faktor VIII-Aktivität enthalten Faktor IXa und Faktor X sowie nur geringe Mengen an Thrombin, sind aber dennoch zur Herstellung eines erfindungsgemäßen Testreagens geeignet.

Da die oben beschriebenen gereinigten Blutfaktoren zur Testlösung zugegeben werden, kann der Test durchgeführt werden, ohne daß man sich auf die ganze Koagulationskaskade zu stützen braucht. Dies ermöglicht einen einfacheren und verläßlicheren Test und ermöglicht die Verwendung geringerer Konzentrationen der Testprobe. Eine Verringerung der im Test verwendeten Testprobenmengen reduziert die von bestimmten Blutfaktoren in der Probe ausgeübten Störwirkungen auf ein Minimum oder eliminiert diese.

Ein weiterer Vorteil des vorliegenden Tests ist, daß die Ergebnisse nicht durch eine Antikoagulationstherapie mit Substanzen, wie Coumarin und Heparin, die in Proben von Patienten vorkommen können, die Tests unterzogen werden, beeinflußt werden. Dieser Vorteil ist wichtig bei der Untersuchung und Überwachung von Patienten, die wegen thromboembolischer Erkrankungen eine Antikoagulationstherapie durchmachen. Eine genaue Diagnose und Überwachung ihres APC-Empfindlichkeitsstatus erfordert, daß die Antikoagulationstherapie auf die Testergebnisse keine unerwünschte Wirkung hat.

Die im erfindungsgemäßen Test zu verwendende Menge an APC sollte etwa 50% des in der Testprobe vorhandenen Faktor VIIIa abbauen. Ein Bereich zwischen 0,05 bis 5 E/ml APC ist bevorzugt, wobei der Bereich zwischen 0,2 und 0,4 E/ml am meisten bevorzugt ist. Die Menge an in einer Testprobe vorhandenem Faktor VIII kann mittels einer Vielfalt von Mitteln, wie der Kontrolle des vorliegenden Tests, bestimmt werden. Die Menge an Faktor VIII in der Testprobe beeinflußt die zu verwendende Menge an APC. Die Menge an im Test verwendetem Faktor X sollte etwa 0,01 bis 10 E/ml sein. Eine bevorzugte Konzentration von Faktor IXa beim erfindungsgemäßen Verfahren liegt im Bereich zwischen 0,05 und 5 E/ml; Thrombin wird bevorzugt in einer Konzentration von 0,01 bis 2,0 E/ml eingesetzt. Ein bevorzugter Konzentrationsbereich von Phospholipiden liegt beim erfindungsgemäßen Verfahren zwischen 0,01 und 100 nmol/ml, jener von Calciumionen zwischen 1 und 50 µmol/ml.

Ein Tetrapeptid (AcOH-Gly-Pro-Arg-Pro-OH) wird oft zum Reagens zugegeben, um die Gerinnselbildung während des Tests zu verhindern. Ein Heparinneutralisierungsmittel, wie Polybren, kann auch im Test verwendet werden.

Gemäß einer weiteren Ausführungsform der Erfindung enthält die Testlösung weiters Protein S. Damit ist gewährleistet, daß Protein S in der Testlösung im Überschuß vorliegt. Dabei wird die Protein S-Konzentration so gewählt, daß ein weiterer Zusatz von Protein S zur Testlösung keine veränderte "APC response" hervorruft. Es hat sich herausgestellt, daß Protein S vorteilhafterweise im 1 bis 10fachen Überschuß vorliegen soll. Das bedeutet, daß das Verhältnis von Protein S zur unverdünnten Testprobe im Bereich von 1 bis 10 Einheiten pro ml liegt. Ein besonders bevorzugtes Verhältnis liegt im Bereich von 3 bis 5 Einheiten pro ml unverdünnter Testprobe. Als Protein S wird vorzugsweise freies Protein S verwendet, also jenes Protein, welches nicht mit C4b-bindendem Protein komplexiert ist. Durch den Zusatz des Protein S zum Testsystem wird die Bestimmung der "APC response" unabhängig von einem eventuell reduzierten Protein S-Gehalt der Probe möglich.

Es hat sich nämlich überraschenderweise herausgestellt, daß bei einem zu geringen Protein S-Gehalt in einer Testprobe das Meßergebnis der "APC response" derart verändert wird, daß eine verminderte, pathologische "APC response" vorgetäuscht werden kann. Dies ist vor allem bei Patienten von Bedeutung, die einer Therapie mit oralen Antikoagulantien, wie Coumarin und Warfarin, unterzogen werden. Diese Therapie bringt nämlich in manchen Fällen die Reduktion des Protein S-Gehalts im Blut mit sich.

Der vorliegende Test erfordert keine Zugabe von Faktor VIII. Der Test kann sich auf den normalerweise in der Testprobe vorhandenen Faktor VIII stützen, solange die Probe das 0,3 bis 1,5fache der normalen Menge an Faktor VIII enthält, was über die Kontrolle des vorliegenden Tests oder mit anderen Mitteln bestimmt werden kann. Der in der Testprobe vorhandene Faktor VIII wird durch das im Test zugegebene Thrombin aktiviert. Außerdem sollte die Testprobe oder Testlösung mindestens 50% der nomalen Menge an Protein S enthalten, was über funktionale Tests bestimmt werden kann. Protein S kann in jenen Situationen, in welchen ein Protein S-Mangel bekannt oder vermutet wird, zur Testlösung zugegeben werden, doch ist eine Auswertung anderer Blutfaktoren erwünscht.

Bei einer bevorzugten Ausführungsform umfaßt der Test die Umsetzung der Testprobe mit Faktor X, Faktor IXa (Faktor IXaβ), Thrombin, Calciumionen und Phospholipiden in Gegenwart von APC und in Abwesenheit von APC (Kontrolle). Eine Testphiole, die eine mit Faktor X, Faktor IXa (Faktor IXaβ), Thrombin, Calciumionen, Phospholipiden und APC umgesetzte Testprobe enthält, würde auf Faktor Xa ausgewertet. Diese Ergebnisse würden mit den Ergebnissen aus einer Kontrollphiole verglichen, die eine mit Faktor IXa (Faktor IXaβ), Faktor X, Thrombin, Calciumionen, Phospholipiden ohne die Zugabe von APC umgesetzte Testprobe enthält. Ein Verhältnis auf Basis der Menge an in der Kontrolle gebildetem Faktor Xa geteilt durch die Menge an im Test gebildeten Faktor Xa zeigt die APC-Empfindlichkeit der Testprobe an.

Unabhängig von der Ursache der abnormalen APC-Empfindlichkeit kann der vorliegende Test zur Bestimmung der APC-Wirkung verwendet werden. Beispielsweise kann mittels des vorliegenden Tests eine verringerte APC-Empfindlichkeit selbst dort nachgewiesen werden, wo der APC-resistente Faktor VIIIa vorhanden ist. In einem solchen Fall kann der Faktor VIIIa durch APC nicht abgebaut werden, doch kann der Faktor VIIIa noch immer seine normalen Funktionen der Umwandlung von Faktor X zu Faktor Xa ausüben. Dieser Test ist auch zum Nachweis von Abnormitäten in bezug auf die natürlichen Inhibitoren von APC geeignet.

Die Verwendung des vorliegenden Tests kann durch vorverpackte Testsets und Reagenskomponenten erleichtert werden. Die Faktoren und andere Materialien werden vorzugsweise bis zu einem bestimmten Grad getrennt gelagert. Beispielsweise wurden Mehrfachkomponenten-Testsets für den Test entwickelt, um eine leichte Verwendung und eine maximale Haltbarkeit zu gewährleisten.

Diese Testsets umfassen vorzugsweise zwei oder drei Reagenskomponenten. Testsets mit drei Reagenskomponenten sind bevorzugt, da solche Sets leicht verfügbare Kontrollen ermöglichen, da das APC separat gelagert wird. Beispiele für Formulierungen für Testsets sind nachstehend angeführt.

Der vorliegende Test kann mit einem Testset, das eine erste Reagenskomponente A und eine zweite Reagenskomponente B zur Erzeugung des beschriebenen Reagens umfaßt, ausgeübt werden. Die Reagenskomponente A enthält die Faktoren IXa und X und Calciumionen, und die Reagenskomponente B enthält Thrombin, Phospholipide und APC.

Ein Testset, das eine Reagenskomponente C und eine Reagenskomponente D zur Erzeugung des Reagens umfaßt, kann ebenso verwendet werden. Die Reagenskomponente C enthält die Faktoren IXa und X, Calciumionen und Thrombin, und die Reagenskomponente D enthält Phospholipide und APC.

Ebenso kann das erfindungsgemäß verwendbare Testset zur Bestimmung der Empfindlichkeit einer Testprobe gegenüber aktiviertem Protein C Protein S, vorzugsweise freies Protein S, enthalten. Dabei kann Protein S entweder in der Reagenskomponente enthalten sein, die weiters Faktor IXa und Faktor X und gegebenenfalls Calciumionen enthält, oder in der Reagenskomponente mit aktiviertem Protein C.

Der vorliegende Test ist auch für ein Testset mit drei Komponenten geeignet. Testsets mit drei Komponenten, die APC als einzelne Komponente haben, werden bevorzugt, weil das Set dadurch leicht zur Auswertung einer Testprobe und einer Kontrollprobe adaptiert werden kann. Da APC in einer eigenen Phiole vorhanden wäre, kann eine Kontrolle dadurch vorgenommen werden, daß das APC (Reagenskomponente) durch ein gleiches Volumen Puffer ersetzt wird.

Ein Drei-Komponenten-Set umfaßt eine erste Reagenskomponente E, eine zweite Reagenskomponente F und eine dritte Reagenskomponente G zur Erzeugung des beschriebenen Reagens. Die Reagenskomponente E enthält die Faktoren IXa und X und Calciumionen. Die Reagenskomponente F enthält Thrombin und Phospholipide. Die Reagenskomponente G enthält APC.

Beim am meisten bevorzugten Drei-Komponenten-Set sind auch die Phospolipide von allen anderen Materialien getrennt. Diese Trennung ergibt eine weitere Verbesserung der Stabilität der Reagenskomponenten. Das bevorzugte Drei-Komponenten-Set umfaßt eine erste Regenskomponente H, eine zweite Reagenskomponente I und eine dritte Reagenskomponente J zur Herstellung des beschriebenen Reagens. Die Reagenskomponente H enthalt die Faktoren IXa und X, Calciumionen und Thrombin. Die Reagenskomponente I enthält Phospholipide. Die Reagenskomponente J enthält APC.

Bevorzugt wird Faktor IXaß im Reagens verwendet, weil er ein stabiles Reagens gewährleistet. Faktor IXaß kann aus Humanplasma mittels Standardtechniken unter Verwendung von Celite® (bestehend aus Kieselgur; John-Manville Corp.) erhalten werden. Die Anwesenheit der zusätzlichen Koagulationsfaktoren XIa und XIIa kann auch zur Aufrechterhaltung der Faktor IXa-Aktivität nützlich sein.

Das folgende Beispiel enthält Ergebnisse aus einer APC-Empfindlichkeitsbestimmung in verschiedenen Testproben.

### Beispiel 1. APC-Empfindlichkeitstest mit einem Drei-Komponenten-Testset

Es wurde das folgende Testset verwendet:
100 µl Reagenskomponente H: Faktor IXaβ, Faktor X, Calciumionen, Thrombin und Albumin
100 µl Reagenskomponente I: Phospholipide und Albumin
50 µl Reagenskomponente J: aktiviertes Protein C (APC) in einer Konzentration von 2E/ml.

Die Bestandsmaterialien jeder Reagenskomponente können gemäß der EP-A-0496 723 und der EP-A-0 519 903 hergestellt werden. Andere Methoden zur Herstellung dieser Bestandsmaterialien sind wohlbekannt. Man kann diese Bestandsmaterialien auch im Handel erwerben. Beispielsweise sind die Komponenten H und I unter dem Namen IMMUNOCHROM® FVIII:C, IMMUNO AG, im Handel erhältlich.

Die Reagenskomponenten werden mit 50 µl einer Plasmaprobe (1:20 verdünnt) gemischt und 5 Minuten lang bei 37°C inkubiert. Der erzeugte Faktor Xa wird dann unter Verwendung von 200 µl einer verdünnten chromogenen Substratlösung (4 mmol/l CH₃OCO-D-CHA-GLY-ARG-pNA-AcOH gemäß der Instruktionen von IMMUNOCHROM® F VIII: C, IMMUNO AG) bestimmt. Diese Lösung enthält auch α-NAPAP (Nα-(2-Naphthalensulfonylglycyl)-4-amidino-DL-phenylalaninpiperidin) zur Inhibition von Thrombin, und EDTA, um eine weitere Aktivierung von Faktor X zu stoppen. Nach 5minütiger Inkubation wurde die Reaktion mit 100 µl Essigsäure (50% in Wasser V/V) für eine Endauswertung des Verfahrens gestoppt. Die Lichtabsorption wurde bei 405 nm gemessen, um die Menge an während des Tests gebildeten Faktor Xa anzuzeigen. Im Kontrollexperiment wird die Plasmaprobe nur mit den Reagenskomponenten H und I und 50 µl Puffer inkubiert. Die Kontrolle enthält kein zugesetztes APC. Es wird das Verhältnis der Absorption der beiden Testmischungen (ohne und mit APC) errechnet.

### Beispiel 2. Testen der Proben

Die folgenden Testproben wurden mit dem Drei-Komponenten-Testset aus Beispiel 1 getestet:
(1) Citrat-versetztes Normalplasma - C-npl
- Lyophilisiertes Referenzplasma 100% (IMMUNO AG, Wien)
- Gefrorenes normales Kontrollplasma (George King, US)
- Gefrorene Plasmaproben von gesunden Probanden (40-120% der normalen Faktor VIII-Mengen)

(2) Heparinisiertes Normalplasma - H-npl
- Heparin-Kontrollplasma (IMMUNO AG, Wien)

(3) APC-resistentes Plasma mit normalem Protein S-Gehalt:
- Kontrollplasma-Level 2 (Copl2) aus dem "COATEST APC resistance kit" (Chromogenix, Schweden)

(4) Patientenplasma (P), diese Patienten waren thrombophil, haben aber normale Protein C- und Protein S-Mengen.

| **ERGEBNISSE** | |
|---|---|
| Probe | Verhältnis (Mittel ± SD) |
| C-npl | 1,85 ± 0,09 |
| H-npl | 1,8 |
| Copl2 | 1,49 ± 0,04 * |
| P | 1,51 ± 0,04 * |

| | |
|---|---|
| * - wiederholte Versuche an derselben Plasmaprobe | |

Aufgrund der obigen Versuche ist der Verhältnisbereich von Plasma mit normaler APC-Empfindlichkeit 1,65-2,05 (Mittel ± SD). Werte, die unterhalb dieses Bereichs liegen, zeigen eine verringerte APC-Empfindlichkeit an. Werte, die über diesem Bereich liegen, zeigen eine erhöhte APC-Empfindlichkeit an.

Selbstverständlich sind die Beschreibung und die hier angeführten Beispiele, während sie bevorzugte Ausführungsformen der vorliegenden Erfindung anzeigen, nur zur Illustration und beschränken die vorliegende Erfindung nicht.

## Patentansprüche

1. Verfahren zur Bestimmung der Empfindlichkeit gegenüber aktiviertem Protein C in einer Testprobe, welches die Schritte
(a) der Umsetzung von Faktor X, aktiviertem Faktor IX, aktiviertem Protein C und Thrombin mit der Testprobe in einer Calciumionen und Phospholipide enthaltenden Testlösung, wobei die Testprobe höchstens 10% V/V der Testlösung ausmacht,
(b) der Bestimmung der Menge an in der Reaktion von Schritt (a) gebildetem aktiviertem Faktor X,
(c) der Berechnung der Empfindlichkeit der Testprobe gegen-über aktiviertem Protein C auf Basis der Bestimmung von Schritt (b) im Vergleich zu einer Kontrolle umfaßt.

2. Verfahren nach Anspruch 1, wobei die Kontrolle eine durch Umsetzung der Testprobe mit Faktor X, aktiviertem Faktor IX und Thrombin in Anwesenheit von Calciumionen und Phospholipiden ohne Zugabe von aktiviertem Protein C hergestellte Kontrollösung und die Bestimmung der Menge an dadurch gebildetem aktiviertem Faktor X umfaßt.

3. Verfahren nach einem der Ansprüche 1 oder 2, wobei der Faktor IXa Faktor IXaβ ist.

4. Verfahren nach einem der Ansprüche 1 bis 3, wobei der Bestimmungsschritt durch Zugabe eines chromogenen Substrats durchgeführt wird.

5. Verfahren nach einem der Ansprüche 1 bis 4, wobei die Konzentration von Faktor X 0,01-10 E/ml und die Konzentration von aktiviertem Protein C 0,05-5 E/ml beträgt.

6. Verfahren nach einem der Ansprüche 1 bis 5, wobei die Konzentration von Faktor IXa 0,05-5 E/ml und die Konzentration von Thrombin 0,01-2,0 E/ml beträgt.

7. Verfahren nach einem der Ansprüche 1 bis 6, wobei die Konzentration der Phospholipide 0,01-100 nmol/ml und die Konzentration der Calciumionen 1-50 µmol/ml beträgt.

8. Verfahren nach einem der Ansprüche 1-7, wobei die Testprobe weniger als 2% V/V der Testlösung ist.

9. Verfahren nach einem der Ansprüche 1-8, wobei die Testprobe 0,8-1,6% V/V der Testlösung ist.

10. Verfahren nach einem der Ansprüche 1 bis 9, **dadurch gekennzeichnet, daß** eine Testlösung verwendet wird, die zusätzlich Protein S enthält.

11. Verfahren nach Anspruch 10, **dadurch gekennzeichnet, daß** eine Testlösung verwendet wird, die freies Protein S enthält.

12. Verfahren nach Anspruch 10 oder 11, **dadurch gekennzeichnet, daß** eine Testlösung verwendet wird, die von einem Patienten stammt, der mit oralen Antikoagulantien behandelt wurde.

13. Verfahren nach einem der Ansprüche 10 bis 12, **dadurch gekennzeichnet, daß** die Konzentration an Protein S entsprechend dem Verhältnis von 1 bis 10 Einheiten, vorzugsweise 3 bis 5 Ein-heiten, pro ml unverdünnter Testprobe gewählt wird.

## Claims

1. A method of determining the sensitivity relative to activated protein C in a test sample, comprising the steps of
(a) reacting factor X, activated factor IX, activated protein C and thrombin with the test sample in a test solution containing calcium ions and phospholipids, the test sample constituting 10% V/V of the test solution at the most,
(b) determining the amount of activated factor X formed in the reaction of step (a),
(c) calculating the sensitivity of the test sample relative to activated protein C on the basis of the determination of step (b), as compared to a control.

2. A method according to claim 1, wherein the control comprises a control solution prepared by reacting the test sample with factor X, activated factor IX and thrombin in the presence of calcium ions and phospholipids without the addition of activated protein C, and the determination of the amount of activated factor X formed thereby.

3. A method according to one of claims 1 or 2, wherein the factor Ixa is factor IXaβ.

4. A method according to any one of claims 1 to 3, wherein the determination step is carried out by the addition of a chromogenic substrate.

5. A method according to any one of claims 1 to 4, wherein the concentration of factor X is 0.01-10 U/ml, and the concentration of activated protein C is 0.05-5 U/ml.

6. A method according to any one of claims 1 to 5, wherein the concentration of factor IXa is 0.05-5 U/ml, and the concentration of thrombin is 0.01 to 2.0 U/ml.

7. A method according to any one of claims 1 to 6, wherein the concentration of the phospholipids is 0.01-100 nmol/ml, and the concentration of the calcium ions is 1-50 µmol/ml.

8. A method according to any one of claims 1 to 7, wherein the test sample is less than 2% V/V of the test solution.

9. A method according to any one of claims 1 to 8, wherein the test sample is 0.8-1.6% V/V of the test solution.

10. A method according to any one of claims 1 to 9, **characterised in that** a test solution is used which additionally contains protein S.

11. A method according to claim 10, **characterised in that** a test solution is used which contains free protein S.

12. A method according to claim 10 or 11, **characterised in that** a test solution is used which is derived from a patient who has been treated with oral anticoagulants.

13. A method according to any one of claims 10 to 12, **characterised in that** the concentration of protein S is selected corresponding to a ratio of 1 to 10 units, preferably 3 to 5 units, per ml of undiluted test sample.

## Revendications

1. Procédé de détermination de la sensibilité à la protéine C activée dans un échantillon à tester, comprenant les étapes suivantes :
(a) la mise à réagir de facteur X, de facteur IX activé, de protéine C activée et de thrombine avec l'échantillon à tester dans une solution de test contenant des ions de calcium et des phospholipides, l'échantillon à tester représentant au maximum 10% V/V de la solution de test,
(b) la détermination de la quantité de facteur X activé formé au cours de la réaction de l'étape (a),
(c) le calcul de la sensibilité de l'échantillon à tester à la protéine C activée sur la base de la détermination de l'étape (b) par comparaison avec un contrôle.

2. Procédé selon la revendication 1, dans lequel le contrôle comprend une solution de contrôle préparée en faisant réagir l'échantillon à tester avec du facteur X, du facteur IX activé et de la thrombine en présence d'ions de calcium et de phospholipides sans ajout de protéine C activée et en déterminant la quantité de facteur X activé ainsi formé.

3. Procédé selon la revendication 1 ou la revendication 2, dans lequel le facteur IXa est le facteur IXaβ.

4. Procédé selon l'une des revendications 1 à 3, dans lequel l'étape de détermination se fait par ajout d'un substrat chromogène.

5. Procédé selon l'une des revendications 1 à 4, dans lequel la concentration en facteur X est de 0,01 à 10 U/ml et la concentration en protéine C activée est de 0,05 à 5 U/ml.

6. Procédé selon l'une des revendications 1 à 5, dans lequel la concentration en facteur IXa est de 0,5 à 5 U/ml et la concentration en thrombine est de 0,01 à 2,0 U/ml.

7. Procédé selon l'une des revendications 1 à 6, dans lequel la concentration en phospholipides est de 0,01 à 100 nmol/ml et la concentration en ions de calcium est de 1 à 50 µmol/ml.

8. Procédé selon l'une des revendications 1 à 7, dans lequel l'échantillon à tester représente moins de 2% V/V de la solution de test.

9. Procédé selon l'une des revendications 1 à 8, dans lequel l'échantillon à tester représente de 0,8 à 1,6% V/V de la solution de test.

10. Procédé selon l'une des revendications 1 à 9, **caractérisé en ce que** l'on utilise une solution de test qui contient en plus de la protéine S.

11. Procédé selon la revendication 10, **caractérisé en ce que** l'on utilise une solution de test qui contient de la protéine S libre.

12. Procédé selon la revendication 10 ou la revendication 11, **caractérisé en ce que** l'on utilise une solution de test provenant d'un patient qui a été traité par voie orale avec des anticoagulants.

13. Procédé selon l'une des revendications 10 à 12, **caractérisé en ce que** la concentration en protéine S est choisie selon un rapport de 1 à 10 unités, de préférence de 3 à 5 unités, par ml d'échantillon à tester non dilué.
